# EUROPÄISCHE PATENTSCHRIFT

(11) **EP 3 328 350 B1**
(45) Veröffentlichungstag und Bekanntmachung des Hinweises auf die Patenterteilung: **11.09.2019**
(21) Anmeldenummer: 16736165.8
(22) Anmeldetag: 07.07.2016
(51) Int. Cl.: A61K 8/34, A61K 8/40, A61K 8/55, A61Q 17/04

(54) **GERUCHSSTABILE OCTOCRYLEN ENTHALTENDE ZUBEREITUNG**
PREPARATION CONTAINING ODOUR-STABLE OCTOCRYLENE
PRÉPARATION CONTENANT DE L'OCTOCRYLÈNE À ODEUR STABLE

(30) Priorität: 30.07.2015 DE 102015214499
(43) Veröffentlichungstag der Anmeldung: 06.06.2018
(73) Patentinhaber: Beiersdorf AG, 20253 Hamburg (DE)
(72) Erfinder: WEINERT, Katrin, 22763 Hamburg (DE); BORCHERS, Kathrin, 21614 Buxtehude (DE); BLECKMANN, Andreas, 22926 Ahrensburg (DE); PASTERNAK, Anja, 22085 Hamburg (DE)
(86) Internationale Anmeldenummer: PCT/EP2016/066116
(87) Internationale Veröffentlichungsnummer: WO 2017/016840

(56) Entgegenhaltungen:
- EP-A1- 2 243 465
- EP-A2- 0 781 804
- EP-A2- 2 002 822
- WO-A1-87/02714
- US-A1- 2006 104 923

## Beschreibung

Die vorliegende Erfindung betrifft eine kosmetische Zubereitung, enthaltend 2-Ethylhexyl-2-cyano-3,3-diphenylacrylat (Octocrylen), Ethanol und Laurylalkoholdiphosphonsäure.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurden daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie dem Anlage 7 der Kosmetikverordnung zusammengefasst.

Die Vielzahl an kommerziell erhältlichen Sonnenschutzmitteln darf jedoch nicht darüber hinwegtäuschen, dass diese Zubereitungen des Standes der Technik eine Reihe von Nachteilen aufweisen.

Ein vielfach in Tagespflegeprodukten und Sonnenschutzmitteln eingesetzter UV-Filter ist die Verbindung 2-Ethylhexyl-2-cyano-3,3-diphenylacrylat (Octocrylen), die neben ihren UV-Filtereigenschaften im UV-B-Bereich vor allem dazu verwendet wird den UV-A-Filter 4-(tert.-Butyl)-4'-methoxydibenzoylmethan gegen photochemischen Abbau zu stabilisieren.

Nachteilig am Stande der Technik ist dabei der Umstand, dass Octocrylen in Gegenwart von Ethanol in der Regel dazu führt, dass bei den Zubereitungen im Verlauf ihrer Lagerung sowie bei der Anwendung auf der Haut nach einer gewissen Zeit ein "Fehlgeruch" auftritt. Der entstehende Geruch erinnert entfernt an die aus der Küche bekannte Maggi-Stammwürze oder auch an den Geruch von Liebstöckel. Ursache für diesen Fehlgeruch ist die Bildung von Sotolon (3-Hydroxy-4,5-dimethyl-5H-furan-2-on), welches die folgende Struktur aufweist:

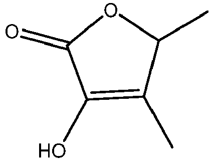

Der Reaktionsmechanismus, welcher zur Entstehung von 3-Hydroxy-4,5-dimethyl-5H-furan-2-on (im Folgenden Sotolon genannt) führt, ist bis heute unbekannt und unverstanden.

Eine Möglichkeit, den Fehlgeruch zu vermeiden, besteht natürlich darin, auf den Einsatz von Ethanol zu verzichten. Ethanol wird jedoch zunehmend dazu verwendet, die bei den Verbrauchern so beliebten "Konservierungsmittel freien" kosmetischen Zubereitungen vor mikrobiellem Befall zu schützen. Darüber hinaus bildet Ethanol aufgrund seiner Lösungseigenschaften, seiner sensorischen Eigenschaften sowie seines geringen Preises die ideale Grundlage für transparente Sprayzubereitungen, beispielsweise Sonnenschutzsprays.

Nach dem Stand der Technik versucht man daher bei ethanolischen Zubereitungen, die geruchliche Fehlnote des Sotolons durch den Einsatz entsprechend hoher Mengen an Parfümstoffen zu maskieren. Diese Lösung hat jedoch den Nachteil, dass sich die Parfümstoffe im Laufe der Zeit aus der Zubereitung heraus verflüchtigen, während die Konzentration an Sotolon im Verlaufe der Zeit ansteigt. Darüber hinaus gibt es auch bei Kosmetika einen wachsenden Bedarf an sogenannten "parfümfreien" Zubereitungen, bei denen eine Geruchsmaskierung dann nicht möglich ist.

Es war daher die Aufgabe der vorliegenden Erfindung, eine kosmetische Zubereitung (insbesondere ein Tagespflegeprodukt oder ein Sonnenschutzmittel) enthaltend Octocrylen und Ethanol zu entwickeln, bei dem die Bildung eines Fehlgeruches und insbesondere die Bildung des Sotolons unterdrückt wird.

Überraschend gelöst wird die Aufgabe durch eine kosmetische Zubereitung, enthaltend
a) 2-Ethylhexyl-2-cyano-3,3-diphenylacrylat (Octocrylen)
b) Ethanol und
c) Laurylalkoholdiphosphonsäure.

Dabei umfasst der Begriff "Laurylalkoholdiphosphonsäure" erfindungsgemäß auch die Salze (insbesondere Mono- und Di- Alkalisalze) dieser Säure.

Laurylalkoholdiphosphonsäure weist dabei die folgende Struktur auf:

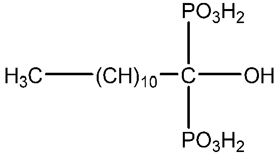

Es ist erfindungsgemäß vorteilhaft, wenn die Zubereitung 2-Ethylhexyl-2-cyano-3,3-diphenyl-acrylat (Octocrylen) in einer Gesamtmenge von 0,1 bis 10 Gewichts-%, bezogen auf das Gesamtgewicht der Zubereitung, enthält. Erfindungsgemäß bevorzugt ist dabei ein Gehalt von 2-Ethylhexyl-2-cyano-3,3-diphenylacrylat (Octocrylen) in einer Gesamtmenge von 4 bis 10 Gewichts-%, bezogen auf das Gesamtgewicht der Zubereitung.

Erfindungsgemäß vorteilhafte Ausführungsformen im Sinne der vorliegenden Erfindung sind dadurch gekennzeichnet, dass die Zubereitung Ethanol in einer Gesamtmenge von 0,1 bis 65 Gewichts-%, bezogen auf das Gesamtgewicht der Zubereitung, enthält. Dabei ist eine Gesamtmenge von 3 bis 60 Gewichts-% Ethanol, bezogen auf das Gesamtgewicht der Zubereitung, erfindungsgemäß bevorzugt.

Es ist erfindungsgemäß von Vorteil, wenn die erfindungsgemäße Zubereitung Laurinsäure enthält.
In einem solchen Fall ist es erfindungsgemäß vorteilhaft, wenn das Gewichtsverhältnis von Laurylalkoholdiphosphonsäure zu Laurinsäure von 1:1 bis 1:10 beträgt.

Derartige Mischungen von Laurylalkoholdiphosphonsäure und Laurinsäure werden beispielsweise von der Firma Bozzetto Group unter dem Handelsnamen Lipokel angeboten. Der Hersteller weist in seinen Produktbroschüren darauf hin, dass dieser Rohstoff Öle vor Oxidation schützen und somit das Ranzig-werden von Zubereitungen inhibieren kann.

Da die Entstehung von Sotolon in den Zubereitungen jedoch nicht allein auf den Gehalt von Octocrylen zurück zu führen ist, sondern in der Gegenwart von Ethanol auftritt, konnte dieser Hinweis nicht den Weg zur vorliegenden Erfindung weisen. Wie sich schon aus der Struktur des Octocrylens und des Sotolons ergibt, kann ein oxidativer Abbau, wie er dem Ranzig-werden zu Grunde liegt, die Entstehung des Sotolons jedenfalls nicht erklären bzw. nahelegen.

Ferner kennt der Stand der Technik die Dokumente EP2243465 A1, US 200671P4923 A1, EP 2002822 A2, WO 87/02714 A1 und EP0781804 A2, die ebenfalls nicht den Weg zur vorliegenden Erfindung weisen konnten.

Erfindungsgemäß vorteilhafte Ausführungsformen der vorliegenden Erfindung sind dadurch gekennzeichnet, dass die Gesamtmenge von Laurylalkoholdiphosphonsäure und Laurinsäure in der Zubereitung von 0,0015 bis 0,2 Gewichts-%, bezogen auf das Gesamtgewicht der Zubereitung, beträgt.
Dabei ist es erfindungsgeimäß bevorzugt, wenn die Gesamtmenge von Laurylalkoholdiphosphonsäure und Laurinsäure in der Zubereitung von 0,015 bis 0,15 Gewichts-%, bezogen auf das Gesamt-gewicht der Zubereitung, beträgt.

Es ist erfindungsgemäß von Vorteil, wenn die Zubereitung Laurylalkoholdiphosphonsäure in einer Gesamtmenge von 0,001 bis 0,15 Gewichts-%, bezogen auf das Gesamtgewicht der Zubereitung enthält.
Es ist erfindungsgemäß bevorzugt, wenn die Zubereitung Laurylalkoholdiphosphonsäure in einer Gesamtmenge von 0,01 bis 0,1 Gewichts-%, bezogen auf das Gesamtgewicht der Zubereitung enthält

Es ist erfindungsgemäß von Vorteil, wenn die Zubereitung Laurinsäure in einer Gesamtmenge von 0,00015 bis 0,05 Gewichts-%, bezogen auf das Gesamtgewicht der Zubereitung enthält.
Es ist erfindungsgemäß bevorzugt, wenn die Zubereitung Laurinsäure in einer Gesamtmenge von 0,0025 bis 0,03 Gewichts-%, bezogen auf das Gesamtgewicht der Zubereitung enthält.

Die erfindungsgemäße Zubereitung kann vorteilhaft in zwei unterschiedlichen Ausführungsformen vorliegen: Als klare (transparente) ethanolische Lipidlösung sowie als Emulsion.

### 1) klare ethanolische Lipidlösung

Diese erfindungsgemäß vorteilhafte Ausführungsformen der vorliegenden Erfindung sind dadurch gekennzeichnet, dass die Zubereitung transparent ist.

Dabei gift eine Zubereitung erfindungsgemäß als transparent, wenn es möglich ist bei Tageslicht durch eine mit der erfindungsgemäßen Zubereitung gefüllten Einmal-Küvette (Firma Brand, 2,5ml, Wellenlängenbereich: 220nm-900nm) mit dem bloßen Auge zu schauen. Schriftzeichen. (Schrifttyp Arial Schriftgröße 10), die sich unmittelbar hinter der Einmal-Küvette befinden, sollten erkennbar und lesbar sein.

Diese erfindungsgemäß vorteilhaften Ausführungsformen der vorliegenden Erfindung sind dadurch gekennzeichnet, dass die Zubereitung weniger als 1 Gewichts-% Wasser, bezogen auf das Gesamtgewicht der Zubereitung, enthält.

### 2) Emulsion

Diese erfindungsgemäß vorteilhafte Ausführungsform liegt bevorzugt in Form einer O/W-Emulsion vor.

Darüber hinaus sind diese Emulsionen als erfindungsgemäß vorteilhaft dadurch gekennzeichnet, dass die Zubereitung einen oder mehrere Emulgatoren gewählt aus der Gruppe der Verbindungen Glycerylstearatcitrat, Cetearylalkohol, Natriumcetearylsulfat + Glycerylstearat, Cetearylsulfosuccinat, Natriumstearoylglutamat, Polyglyceryl-3-methylglucosedistearat, Stearinsäure, Polyglyceryl-10 Stearat, Polyglyceryl-4 Diisostearate/Polyhydroxystearate/Sebacate Kaliumcetylphosphat, enthält.
Die Emulgatorkonzentration (Gesamtkonzentration) beträgt dabei vorteilhaft von 0,1 bis 3% Gewichts-%, bezogen auf das Gesamtgewicht der Zubereitung.

Vorteilhaft im Sinne der vorliegenden Erfindung ist es nicht zuletzt, wenn der Wassergehalt der o/w Emulsionen von 20 bis 60 Gewichts-%, bezogen auf das Gesamtgewicht der Zubereitung beträgt.

Beide erfindungsgemäß vorteilhaften Ausführungsformen der vorliegenden Erfindung sind dadurch gekennzeichnet, dass die Zubereitung einen oder mehrere UV-Filter enthält, gewählt aus der Gruppe der Verbindungen 2-Phenylbenzimidazol-5-sulfonsäure und/oder deren Salze; Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 4-(tert.-Butyl)-4'-methoxydibenzoylmethan; Homomenthylsalicylat (Homosalate); 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan / Dimethylsiloxan - Copolymer; 4-(tert.-Butyl)-4'-methoxydibenzoylmethan; 2-(4'-Diethylamino-2'-hydoxybenzoyl)-benzoesäurehexylester; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin); 4,4',4"-(1,3,5-Triazin-2,4,6-triyltriimino)-tris-benzoësäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; Merocyanine; Titandioxid; Zinkoxid.

Dabei ist es erfindungsgemäß bevorzugt, wenn die Zubereitung Homomenthylsalicylat (Homosalate) und/oder Ethylhexylsalicylat enthält.

Enthält die erfindungsgemäße Zubereitung Homomenthylsalicylat (Homosalate), so ist es erfindungsgemäß von Vorteil, diesen Stoff in einer Konzentration von 0,1 bis 15 Gewichts-%, bezogen auf das Gesamtgewicht der Zubereitung einzusetzen.

Enthält die erfindungsgemäße Zubereitung Ethylhexylsalicylat, so ist es erfindungsgemäß von Vorteil, diesen Stoff in einer Konzentration von 0,1 bis 5 Gewichts-%, bezogen auf das Gesamtgewicht der Zubereitung einzusetzen.

Enthält die erfindungsgemäße Zubereitung 4-(tert.-Butyl)-4'-methoxydibenzoylmethan, so ist es erfindungsgemäß von Vorteil, diesen Stoff in einer Konzentration von 0,1 bis 5 Gewichts-%, bezogen auf das Gesamtgewicht der Zubereitung einzusetzen.

Erfindungsgemäß vorteilhafte Ausführungsformen der vorliegenden Erfindung sind dadurch gekennzeichnet, dass die Zubereitung frei ist von Propyl- und Butylparaben, 3-lod-2-propinylbutylcarbamat, 3-(4-Methylbenzyliden)-campher und 2-Hydroxy-4-methoxybenzophenon (Oxybenzon).

Es ist vorteilhaft im Sinne der vorliegenden Erfindung, wenn die Zubereitung C12-15 Alkylbenzoat, Cyclomethicon, Octyldodecanol und/oder Capryl/Caprinsäure Triglyceride enthält.

Enthält die erfindungsgemäße Zubereitung C12-15 Alkylbenzoat, so ist es erfindungsgemäß von Vorteil, diesen Stoff in einer Konzentration von 0,1 bis 15 Gewichts-%, bezogen auf das Gesamtgewicht der Zubereitung einzusetzen.

Enthält die erfindungsgemäße Zubereitung Cyclomethicon, so ist es erfindungsgemäß von Vorteil, diesen Stoff in einer Konzentration von 0,1 bis 10 Gewichts-%, bezogen auf das Gesamtgewicht der Zubereitung einzusetzen.

Enthält die erfindungsgemäße Zubereitung Capryl/Caprinsäure Triglyceride, so ist es erfindungsgemäß von Vorteil, diesen Stoff in einer Konzentration von 0,1 bis 25 Gewichts-%, bezogen auf das Gesamtgewicht der Zubereitung einzusetzen.

Darüber hinaus kann die lipophile Phase der erfindungsgemäßen Zubereitung weitere lipophile Inhaltsstoffe enthalten, wie sie üblicherweise in der Kosmetik eingesetzt werden. Erfindungsgemäß vorteilhaft ist beispielsweise auch der Einsatz von Butylenglykol Dicaprylat/Dicaprat, Phenethylbenzoat, Cocoglycerid und Di-n-butyladipat bzw. Diisopropyladipat.

Darüber hinaus ist es erfindungsgemäß vorteilhaft, wenn die Zubereitung als weitere Inhaltsstoffe eine oder mehrere Verbindungen gewählt aus der Gruppe der Verbindungen alpha-Liponsäure, Folsäure, Phytoen, D-Biotin, Coenzym Q10, alpha-Glucosylrutin, Carnitin, Carnosin, Polydocanol, natürliche und/oder synthetische Isoflavonoide, Flavonoide, Kreatin, Kreatinin, Taurin, β-Alanin, Tocopherylacetat, Panthenol, Magnolol, Honokiol, Harnstoff, Hyaluronsäure, Dihydroxyaceton, 8-Hexadecen-1,16-dicarbonsäure, Glycyrrhetinsäure, Glucosylglyceride und/oder Licochalcon A enthält.
Erfindungsgemäß besonders bevorzugt ist dabei der Einsatz von Glycyrrhetinsäure bzw. Glycyrrhetinsäure enthaltender Pflanzenextrakte (z.B. *Glycyrrhiza glabra*).

Es ist vorteilhaft im Sinne der vorliegenden Erfindung, wenn die Zubereitung ein oder mehrere Verdickungsmittel enthält.

Erfindungsgemäß bevorzugt werden dabei ein oder mehrere Verdickungsmittel gewählt aus der Gruppe der Verbindungen Ammonium Acryloyldimethyltaurate/VP Copolymer, Ammonium Acryloyldimethyltaurate/ Beheneth-25 Methacrylate Copolymer, Hydroxypropylcellulose Vinylpyrrolidon/Acrylsäure-Copolymer enthält.

Es ist insbesondere von Vorteil, die Filmbildner aus der Gruppe der Polymere auf Basis von Polyvinylpyrrolidon (PVP) 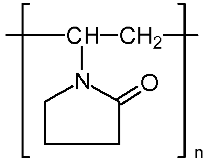 zu wählen. Besonders bevorzugt sind Copolymere des Polyvinylpyrrolidons, beispielsweise das PVP Hexadecen Copolymer und das PVP Eicosen Copolymer, welche unter den Handelsbezeichnungen Antaron V216 und Antaron V220 bei der GAF Chemicals Cooperation erhältlich sind.

Ebenfalls vorteilhaft sind weitere polymere Filmbildner, wie beispielsweise Natriumpolystryrensulfonat, welches unter der Handelsbezeichnung Flexan 130 bei der National Starch and Chemical Corp. erhältlich ist, und/oder Polyisobuten, erhältlich bei Rewo unter der Handelsbezeichnung Rewopal PIB1000. Weitere geeignete Polymere sind z.B. Polyacrylamide (Seppigel 305), Polyvinylalkohole, PVP, PVP / VA Copolymere, Polyglycole, Acrylat/Octylacralymid Copolymer (Dermacryl 79). Ebenfalls vorteilhaft ist die Verwendung von Hydriertem Rizinusöl Dimerdilinoleat (CAS 646054-62-8, INCI Hydrogenated Castor Oil Dimer Dilinoleate), das bei der Firma Kokyu Alcohol Kogyo unter dem Namen Risocast DA-H erworben werden kann oder aber auch PPG-3 Benzylethermyristat (CAS 403517-45-3), das unter dem Handelsnamen Crodamol STS bei der Firma Croda Chemicals erworben werden kann.

Erfindungsgemäß ist auch ein Verfahren zur Geruchsstabilisierung von ethanolhaltigen, kosmetischen Zubereitungen enthaltend 2-Ethylhexyl-2-cyano-3,3-diphenylacrylat (Octocrylen), dadurch gekennzeichnet, dass man der Zubereitung Laurylalkoholdiphosphonsäure zusetzt sowie die Verwendung von Laurylalkoholdiphosphonsäure zur Geruchsstabilisierung von 2-Ethylhexyl-2-cyano-3,3-diphenylacrylat (Octocrylen) enthaltenden ethanolischen kosmetischen Zubereitungen.

### Vergleichsversuche

Mit dem folgenden Vergleichsversuch konnte der erfindungsgemäße Effekt belegt werden:
Es wurde eine Rezeptur mit 0,3 Gew.-% der Substanz Lipokel 12G (enthaltend 10-25 Gew.-% Laurylalkoholdiphosphonsäure sowie 2,5-10 Gew.-% Laurinsäure) von Bozzetto in ein transparentes Sonnenschutzmittel mit einem hohem Ethanol- und einen Octocrylen-Gehalt eingearbeitet (NIVEA sun, Protect & Refresh kühlendes, transparentes Sonnenspray, Artikelnummer 85803 aus dem Jahr 2015) und verglichen mit dem gleichen Produkt ohne Lipokel 12G.

Die geruchliche Veränderung der Muster wurde nach 3, 4 und 5 Monaten im Vergleich zu einem Muster ohne Lipokel 12G bestimmt. Die olfaktorische Beurteilung erfolgte durch einen Geruchsprüfer. Folgende Bewertungsstufen kamen zur Anwendung:
○ : Basisgeruch
+ : keine wahrnehmbare Veränderung
- - : deutliche Würznote (Sotolon)
- : leichte Würznote (Sotolon)

| **Lagerbedingungen** | **Lagerzeitraum** | **Basisprodukt** | **+ 0,3 Gew.-% LIPOKEL 12G** |
|---|---|---|---|
| Frisches Muster | 1 Tag | ○ | ○ |
| Lagerung bei Raumtemperatur | 3. Monat | - | + |
| | 4. Monat | - | + |
| | 5. Monat | - | + |
| Kühlschrank +6°C | 3. Monat | + | + |
| | 4. Monat | - | + |
| | 5. Monat | - | + |
| Brutschrank 40°C | 3. Monat | - | + |
| | 4. Monat | -- | + |
| | 5. Monat | -- | + |
| Lagerung unter Tageslichteinfluss | 3. Monat | -- | + |
| | 4. Monat | -- | + |
| | 5. Monat | -- | + |

### Beispiele

Die nachfolgenden Beispiele sollen die vorliegende Erfindung verdeutlichen ohne sie einzuschränken. Die Angaben beziehen sich stets auf Gewichts-%, sofern nicht andere Angaben gemacht werden.

**1) Klare ethanolische Lipidlösung**

| **Phase** | **INCI** | **Bsp. 1** | **Bsp. 2** | **Bsp. 3** | **Bsp. 4** |
|---|---|---|---|---|---|
| | | **m [%]** | | | |
| A | Butyl Methoxydibenzoylmethane | 4,5 | 4,5 | 4,0 | 4,5 |
| | Bis-Ethylhexyloxyphenol Methoxyphenyl Triazine | | 2,0 | | |
| | Octocrylene | 9,0 | 9,0 | 8,0 | 9,0 |
| | Homosalate | 9,0 | 9,0 | 8,0 | 9,0 |
| | Ethylhexyl Salicylate | 4,5 | 4,5 | 4,0 | 4,5 |
| | C12-15 Alkyl Benzoate | 9,5 | 9,5 | 10,0 | 10,0 |
| | Butylene Glycol Dicaprylate/Dicaprate | | 3,0 | | |
| | Dibutyl Adipate | | 3,0 | | |
| | Caprylic/ Capric Triglyceride | | | 19,0 | 17,0 |
| | Octyldodecanol | | | 19,0 | 17,5 |
| B | Polysilicone-15 | | 1,0 | | |
| | Cyclomethicone | 8,0 | | | |
| | Parfum | q.s. | q.s. | q.s. | q.s. |
| C | Glycerin | 5,0 | 2,5 | | |
| | Acrylates/Octylacrylamide Copolymer | 1,0 | 1,0 | 2,0 | 2,0 |
| | Lipokel 12G (Laurylalkoholdiphosphonsäure, Laurinsäure, Propylenglykol) | 0,3 | 0,5 | 0,3 | 0,3 |
| | Alcohol Denat. | ad. 100 | ad. 100 | ad. 100 | ad. 100 |

### Herstellweise:

1.) Phase A wird unter Rühren auf 85 °C erwärmt und im Anschluss auf Raumtemperatur abgekühlt.
2.) Phase C wird unter Rühren homogen gelöst.
3.) Hinzufügen von Phase B zu Phase A.
4.) Hinzufügen von Phase C.

**2) Emulsionen**

| **INCI** | **Bsp.5** | **Bsp.6** | **Bsp.7** | **Bsp.8** |
|---|---|---|---|---|
| | **m [%]** | | | |
| Ethylhexylglycerin | 0,25 | 0,25 | 0,25 | |
| Phenoxyethanol | | | | 0,50 |
| Glycine | 2,00 | 2,00 | 2,00 | |
| Dibutyl Adipate | 2,50 | 2,50 | 2,50 | |
| Isopropyl Palmitate | 4,00 | 9,00 | 9,00 | 3,00 |
| Dimethicone | 0,30 | 0,30 | 0,30 | |
| Isodecyl Neopentanoate | 7,00 | 8,50 | 8,50 | |
| Butylene Glycol Dicaprylate/Dicaprate | 7,00 | 8,50 | 8,50 | |
| Polyglyceryl-3 Methylglucose Distearate | | | | 0,15 |
| Polybutene | 5,00 | | | |
| Glycerin | 0,50 | | 0,10 | 1,00 |
| Hydroxyethylcellulose | | | | 0,15 |
| Carbomer | | | | 0,15 |
| Acrylates/C10-30 Alkyl Acrylate Crosspolymer | | | | 0,20 |
| Polyglyceryl-4 Diisostearate/Polyhydroxystearate/Sebacate | 1,00 | 1,00 | 1,00 | |
| Methyl Methacrylate Crosspolymer | 5,00 | 5,00 | 5,00 | |
| Polymethylsilsesquioxane | 2,00 | 2,00 | 2,00 | |
| Silica Dimethyl Silylate | 0,50 | 0,50 | 0,50 | 1,00 |
| Tapioca Starch | | | | 3,00 |
| Polyamide-5 | 3,50 | 2,00 | 2,00 | |
| VP/Hexadecene Copolymer | 0,50 | 0,50 | | |
| Alcohol Denat. | 6,00 | 6,00 | 6,00 | 10,00 |
| Trisodium EDTA | 0,20 | 0,20 | 0,20 | 0,20 |
| Homosalate | 2,25 | 2,25 | 2,25 | 9,00 |
| Ethylhexyl Salicylate | 2,25 | 2,25 | 2,25 | 4,50 |
| Octocrylene | 6,00 | 6,00 | 6,00 | 4,50 |
| Bis-Ethylhexyloxyphenol Methoxyphenyl Triazine | 2,25 | 2,25 | 2,25 | |
| Butyl Methoxydibenzoylmethane | 4,50 | 4,50 | 4,50 | 4,50 |
| Titanium Dioxide | 3,00 | 1,50 | | 2,00 |
| Phenylbenzimidazole Sulfonic Acid | 2,50 | 2,50 | 2,50 | |
| Lipokel 12G (Laurylalkoholdiphosphonsäure, Laurinsäure, Propylenglykol) | 0,30 | 0,30 | 0,30 | 0,30 |
| Parfum | q.s. | q.s. | q.s. | q.s. |
| Sodium Hydroxide | q.s. | q.s. | q.s. | q.s. |
| Aqua | ad. 100 | ad. 100 | ad. 100 | ad. 100 |

## Patentansprüche

1. Kosmetische Zubereitung enthaltend
a) 2-Ethylhexyl-2-cyano-3,3-diphenylacrylat (Octocrylen)
b) Ethanol und
c) Laurylalkoholdiphosphonsäure.

2. Kosmetische Zubereitung nach Anspruch 1, **dadurch gekennzeichnet, dass** die Zubereitung
2-Ethylhexyl-2-cyano-3,3-diphenylacrylat (Octocrylen) in einer Gesamtmenge von 0,1 bis 10 Gewichts-%, bezogen auf das Gesamtgewicht der Zubereitung, enthält.

3. Kosmetische Zubereitung nach einem der vorhergehenden Ansprüche, **dadurch gekennzeichnet, dass** die Zubereitung Ethanol in einer Gesamtmenge von 0,1 bis 65 Gewichts-%, bezogen auf das Gesamtgewicht der Zubereitung, enthält.

4. Kosmetische Zubereitung nach einem der vorhergehenden Ansprüche, **dadurch gekennzeichnet, dass** die Zubereitung Laurinsäure enthält.

5. Kosmetische Zubereitung nach Anspruch 4, **dadurch gekennzeichnet, dass** das Gewichtsverhältnis von Laurylalkoholdiphosphonsäure zu Laurinsäure von 1:1 bis 1:10 beträgt.

6. Kosmetische Zubereitung nach einem der vorhergehenden Ansprüche, **dadurch gekennzeichnet, dass** die Gesamtmenge von Laurylalkoholdiphosphonsäure und Laurinsäure in der Zubereitung von 0,0015 bis 0,2 Gewichts-%, bezogen auf das Gesamtgewicht der Zubereitung, beträgt.

7. Kosmetische Zubereitung nach einem der vorhergehenden Ansprüche, **dadurch gekennzeichnet, dass** die Zubereitung Laurylalkoholdiphosphonsäure in einer Gesamtmenge von 0,001 bis 0,15 Gewichts-%, bezogen auf das Gesamtgewicht der Zubereitung, enthält.

8. Kosmetische Zubereitung nach einem der vorhergehenden Ansprüche, **dadurch gekennzeichnet, dass** die Zubereitung transparent ist.

9. Kosmetische Zubereitung nach einem der vorhergehenden Ansprüche, **dadurch gekennzeichnet, dass** die Zubereitung weniger als 1 Gewichts-% Wasser, bezogen auf das Gesamtgewicht der Zubereitung, enthält.

10. Kosmetische Zubereitung nach einem der Ansprüche 1 bis 7, **dadurch gekennzeichnet, dass** die Zubereitung in Form einer Emulsion vorliegt.

11. Kosmetische Zubereitung nach Anspruch 10, **dadurch gekennzeichnet, dass** die Zubereitung einen oder mehrere Emulgatoren gewählt aus der Gruppe der Verbindungen Glycerylstearatcitrat, Cetearylalkohol, Natriumcetearylsulfat + Glycerylstearat, Cetearylsulfosuccinat, Natriumstearoylglutamat, Polyglyceryl-3-methylglucosedistearat, Stearinsäure, Polyglyceryl-10 Stearat, Polyglyceryl-4 Diisostearate/Polyhydroxystearate/Sebacate, Kaliumcetylphosphat, enthält.

12. Kosmetische Zubereitung nach einem der vorhergehenden Ansprüche, **dadurch gekennzeichnet, dass** die Zubereitung einen oder mehrere UV-Filter enthält, gewählt aus der Gruppe der Verbindungen 2-Phenylbenzimidazol-5-sulfonsäure und/oder deren Salze; Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)-benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 4-(tert.-Butyl)-4'-methoxydibenzoylmethan; Homomenthylsalicylat (Homosalate); 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan / Dimethylsiloxan - Copolymer; 4-(tert.-Butyl)-4'-methoxydibenzoylmethan; 2-(4'-Diethylamino-2'-hydoxybenzoyl)-benzoesäurehexylester; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin); 4,4',4"-(1,3,5-Triazin-2,4,6-triyltriimino)-tris-benzoësäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; Merocyanine; Titandioxid; Zinkoxid.

13. Kosmetische Zubereitung nach einem der vorhergehenden Ansprüche, **dadurch gekennzeichnet, dass** die Zubereitung Homomenthylsalicylat (Homosalate) und/oder Ethylhexylsalicylat enthält.

14. Kosmetische Zubereitung nach einem der vorhergehenden Ansprüche, **dadurch gekennzeichnet, dass** die Zubereitung frei ist von Propyl- und Butylparaben, 3-lod-2-propinylbutylcarbamat, 3-(4-Methylbenzyliden)-campher und 2-Hydroxy-4-methoxybenzophenon (Oxybenzon).

15. Kosmetische Zubereitung nach einem der vorhergehenden Ansprüche, **dadurch gekennzeichnet, dass** die Zubereitung C12-15 Alkylbenzoat, Cyclomethicon, Octyldodecanol und/oder Capryl/Caprinsäure Triglyceride enthält.

## Claims

1. Cosmetic preparation comprising
a) 2-ethylhexyl 2-cyano-3,3-diphenylacrylate (octocrylene)
b) ethanol and
c) lauryl alcohol diphosphonic acid.

2. Cosmetic preparation according to Claim 1, **characterized in that** the preparation comprises 2-ethylhexyl 2-cyano-3,3-diphenylacrylate (octocrylene) in a total amount of 0.1 to 10% by weight, based on the total weight of the preparation.

3. Cosmetic preparation according to either of the preceding claims, **characterized in that** the preparation comprises ethanol in a total amount of 0.1 to 65% by weight, based on the total weight of the preparation.

4. Cosmetic preparation according to any of the preceding claims, **characterized in that** the preparation comprises lauric acid.

5. Cosmetic preparation according to Claim 4, **characterized in that** the ratio by weight of lauryl alcohol diphosphonic acid to lauric acid is from 1:1 to 1:10.

6. Cosmetic preparation according to any of the preceding claims, **characterized in that** the total amount of lauryl alcohol diphosphonic acid and lauric acid in the preparation is from 0.0015 to 0.2% by weight, based on the total weight of the preparation.

7. Cosmetic preparation according to any of the preceding claims, **characterized in that** the preparation comprises lauryl alcohol diphosphonic acid in a total amount of 0.001 to 0.15% by weight, based on the total weight of the preparation.

8. Cosmetic preparation according to any of the preceding claims, **characterized in that** the preparation is transparent.

9. Cosmetic preparation according to any of the preceding claims, **characterized in that** the preparation comprises less than 1% by weight water, based on the total weight of the preparation.

10. Cosmetic preparation according to any of Claims 1 to 7, **characterized in that** the preparation is in the form of an emulsion.

11. Cosmetic preparation according to Claim 10, **characterized in that** the preparation comprises one or more emulsifiers selected from the group of compounds comprising glyceryl stearate citrate, cetearyl alcohol, sodium cetearyl sulfate + glyceryl stearate, cetearyl sulfosuccinate, sodium stearoyl glutamate, polyglyceryl-3 methylglucose distearate, stearic acid, polyglyceryl-10 stearate, polyglyceryl-4 diisostearate/polyhydroxystearate/sebacate, potassium cetyl phosphate.

12. Cosmetic preparation according to any of the preceding claims, **characterized in that** the preparation comprises one or more UV filters selected from the group of compounds comprising 2-phenylbenzimidazole-5-sulfonic acid and/or salts thereof; phenylene-1,4-bis(2-benzimidazyl)-3,3'-5,5'-tetrasulfonic acid salts; 1,4-di(2-oxo-10-sulfo-3-bornylidenemethyl)benzene and salts thereof; 4-(2-oxo-3-bornylidenemethyl)benzenesulfonic acid salts; 2-methyl-5-(2-oxo-3-bornylidenemethyl)sulfonic acid salts; 2,2'-methylenebis(6-(2H-benzotriazol-2-yl)-4-(1,1,3,3-tetramethylbutyl)phenol); 2-(2H-benzotriazol-2-yl)-4-methyl-6-[2-methyl-3-[1,3,3,3-tetramethyl-1-[(trimethylsilyl)oxy]disiloxanyl]propyl]phenol; 3-(4-methylbenzylidene)camphor; 3-benzylidenecamphor; ethylhexyl salicylate; terephthalidenedicamphorsulfonic acid; 2-ethylhexyl 4-(dimethylamino)benzoate; amyl 4-(dimethylamino)benzoate; di(2-ethylhexyl) 4-methoxybenzalmalonate; 2-ethylhexyl 4-methoxycinnamate; isoamyl 4-methoxycinnamate; 2-hydroxy-4-methoxybenzophenone, 2-hydroxy-4-methoxy-4'-methylbenzophenone; 2,2'-dihydroxy-4-methoxybenzophenone; 4-(tert-butyl)-4'-methoxydibenzoylmethane; homomenthyl salicylate (homosalate); 2-ethylhexyl 2-hydroxybenzoate; dimethicodiethylbenzalmalonate; 3-(4-(2,2-bis ethoxycarbonylvinyl)phenoxy)propenyl)methoxysiloxane / dimethylsiloxane - copolymer; 4-(tert-butyl)-4'-methoxydibenzoylmethane; hexyl 2-(4'-diethylamino-2'-hydroxybenzoyl)benzoate; dioctylbutylamidotriazone (INCI: Diethylhexyl Butamidotriazone); 2,4-bis[5-1(dimethylpropyl)benzoxazol-2-yl-(4-phenyl)imino]-6-(2-ethylhexyl)imino-1,3,5-triazine with (CAS No. 288254-16-0); 2,4-bis-{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine); tris(2-ethylhexyl) 4,4',4"-(1,3,5-triazine-2,4,6-triyltriimino)trisbenzoate (also: 2,4,6-tris[anilino-(p-carbo-2'-ethyl-1'-hexyloxy)]-1,3,5-triazine (INCI: Ethylhexyl Triazone); 2,4,6-tribiphenyl-4-yl-1,3,5-triazine; merocyanine; titanium dioxide; zinc oxide.

13. Cosmetic preparation according to any of the preceding claims, **characterized in that** the preparation comprises homomenthyl salicylate (homosalate) and/or ethylhexyl salicylate.

14. Cosmetic preparation according to any of the preceding claims, **characterized in that** the preparation is free of propylparaben and butylparaben, 3-iodo-2-propynyl butylcarbamate, 3-(4-methylbenzylidene)camphor and 2-hydroxy-4-methoxybenzophenone (oxybenzone).

15. Cosmetic preparation according to any of the preceding claims, **characterized in that** the preparation comprises C12-15 alkyl benzoate, cyclomethicone, octyldodecanol and/or caprylic/capric acid triglycerides.

## Revendications

1. Préparation cosmétique contenant
a) du 2-cyano-3,3-diphénylacrylate de 2-éthylhexyle (octocrylène)
b) de l'éthanol et
c) de l'acide (1-hydroxy-1,1-dodécanediyl)bis(phosphonique)(« acide diphosphonique de l'alcool laurylique»).

2. Préparation cosmétique selon la revendication 1, **caractérisée en ce que** la préparation contient du 2-cyano-3,3-diphénylacrylate de 2-éthylhexyle (octocrylène) en une quantité totale de 0,1 jusqu'à 10% en poids, par rapport au poids total de la préparation.

3. Préparation cosmétique selon l'une quelconque des revendications précédentes, **caractérisée en ce que** la préparation contient de l'éthanol en une quantité totale de 0,1 jusqu'à 65% en poids, par rapport au poids total de la préparation.

4. Préparation cosmétique selon l'une quelconque des revendications précédentes, **caractérisée en ce que** la préparation contient de l'acide laurique.

5. Préparation cosmétique selon la revendication 4, **caractérisée en ce que** le rapport pondéral d'acide (1-hydroxy-1,1-dodécanediyl)bis(phosphonique) à acide laurique est de 1:1 jusqu'à 1:10.

6. Préparation cosmétique selon l'une quelconque des revendications précédentes, **caractérisée en ce que** la quantité totale d'acide (1-hydroxy-1,1-dodécanediyl)bis(phosphonique) et d'acide laurique dans la préparation est de 0,0015 jusqu'à 0,2% en poids, par rapport au poids total de la préparation.

7. Préparation cosmétique selon l'une quelconque des revendications précédentes, **caractérisée en ce que** la préparation contient de l'acide (1-hydroxy-1,1-dodécanediyl)bis(phosphonique) en une quantité totale de 0,001 jusqu'à 0,15% en poids, par rapport au poids total de la préparation.

8. Préparation cosmétique selon l'une quelconque des revendications précédentes, **caractérisée en ce que** la préparation est transparente.

9. Préparation cosmétique selon l'une quelconque des revendications précédentes, **caractérisée en ce que** la préparation contient moins d'1% en poids d'eau, par rapport au poids total de la préparation.

10. Préparation cosmétique selon l'une quelconque des revendications 1 à 7, **caractérisée en ce que** la préparation se trouve sous forme d'une émulsion.

11. Préparation cosmétique selon la revendication 10, **caractérisée en ce que** la préparation contient un ou plusieurs émulsifiant(s) choisi(s) dans le groupe des composés : stéarate citrate de glycéryle, alcool cétéarylique, cétéarylsulfate de sodium + stéarate de glycéryle, sulfosuccinate de cétéaryle, stéarylglutamate de sodium, méthylglucosedistéarate de polyglycéryle-3, acide stéarique, stéarate de polyglycéryle-10, diisostéarate de polyglycéryle-4/poly(hydroxystéarate)/sébacate, cétylphosphate de potassium.

12. Préparation cosmétique selon l'une quelconque des revendications précédentes, **caractérisée en ce que** la préparation contient un ou plusieurs filtre(s) UV, choisi(s) dans le groupe des composés : acide 2-phénylbenzimidazole-5-sulfonique et/ou ses sels ; sels de l'acide phénylène-1,4-bis(2-benzymidazyl)-3,3'-5,5'-tétrasulfonique ; 1,4-di(2-oxo-10-sulfo-3-bornylidèneméthyl)benzène et ses sels ; sels de l'acide 4-(2-oxo-3-bornylidèneméthyl)benzènesulfonique ; sels de l'acide 2-méthyl-5-(2-oxo-3-bornylidèneméthyl)sulfonique ; 2,2'-méthylène-bis-(6-(2*H-*benzotriazol-2-yl)-4-(1,1,3,3-tétraméthylbutyl)phénol ; 2-(2H-benzotriazol-2-yl)-4-méthyl-6-[2-méthyl-3-[1,1,3,3-tétraméthyl-1-[(triméthylsilyl)oxy]disiloxanyl]propyl]phénol ; 3-(4-méthylbenzylidène)camphre ; 3-benzylidènecamphre ; salicylate d'éthylhexyle ; acide téréphtalidènedicamphresulfonique ; 4-diméthylaminobenzoate de 2-éthylhexyle ; 4-diméthylaminobenzoate d'amyle ; 4-méthoxybenzalmalonate de di(2-éthylhexyle) ; 4-méthoxycinnamate de 2-éthylhexyle ; 4-méthoxycinnamate d'isoamyle ; 2-hydroxy-4-méthoxybenzophénone, 2-hydroxy-4-méthoxy-4'-méthylbenzophénone ; 2,2'-dihydroxy-4-méthoxybenzophénone ; 4-(tert-butyl)-4'-méthoxydibenzoylméthane ; salicylate d'homomenthyle (homosalate) ; 2-hydroxybenzoate de 2-éthylhexyle ; benzalmalonate de diméthicodiéthyle ; copolymère de 3-(4-(2,2-bis-éthoxycarbonylvinyl)-phénoxy)propényl)-méthoxysiloxane/diméthylsiloxane ; 4-(tert-butyl)-4'-méthoxydibenzoylméthane ; 2-(4'-diéthylamino-2'-hydroxybenzoyl)benzoate d'hexyle ; dioctylbutylamidotriazone (INCI (International Nomenclature of Cosmetic Ingredients - nomenclature internationale des ingrédients cosmétiques) : Diethylhexyl-Butamidotriazone) ; 2,4-bis-[5-1(diméthylpropyl)benzoxazol-2-yl-(4-phényl)-imino]-6-(2-éthylhexyl)-imino-1,3,5-triazine (comportant le numéro CAS 288254-16-0) ; 2,4-bis-{[4-(2-éthylhexyloxy)-2-hydroxy]-phényl}-6-(4-méthoxyphényl)-1,3,5-triazine (INCI : Bis-Ethylhexyloxyphenol Methoxyphenyl Triazin) ; 4,4',4"-(1,3,5-triazin-2,4,6-triyltriimino)-tris-benzoate de tris(2-éthylhexyle) (aussi : 2,4,6-tris-[anilino-(p-carbo-2'-éthyl-1'-hexyloxy)]-1,3,5-triazine (INCI : Ethylhexyl Triazone) ; 2,4,6-tribiphényl-4-yl-1,3,5-triazine ; mérocyanine ; dioxyde de titane ; oxyde de zinc.

13. Préparation cosmétique selon l'une quelconque des revendications précédentes, **caractérisée en ce que** la préparation contient du salicylate d'homomenthyle (homosalate) et/ou du salicylate d'éthylhexyle.

14. Préparation cosmétique selon l'une quelconque des revendications précédentes, **caractérisée en ce que** la préparation est exempte de propylparabène et de butylparabène, de 3-iodo-2-propynylbutylcarbamate, de 3-(4-méthylbenzylidène)camphre et de 2-hydroxy-4-méthoxybenzophénone (oxybenzone).

15. Préparation cosmétique selon l'une quelconque des revendications précédentes, **caractérisée en ce que** la préparation contient un benzoate de C₁₂₋₁₅-alkyle, du cyclométicone, de l'octyldodécanol et/ou des triglycérides de l'acide caprylique/caprique.
